# EUROPEAN PATENT APPLICATION

(11) **EP 4 342 363 A1**
(43) Date of publication of application: **27.03.2024**
(21) Application number: 22306399.1
(22) Date of filing: 22.09.2022
(51) Int. Cl.: A61B 5/00, A61B 5/22

(54) **A PORTABLE HANDHELD DEVICE FOR MEASURING THE FORCE OF A JOINT OF A PERSON**

(71) Applicant: Kinvent Biomecanique, 34960 Montpellier (FR)
(72) Inventor: ATHANASSOPOULOS, Emmanouil, 34000 MONTPELLIER (FR); KOLLIAS, Athanase, 34170 CASTELNAU-LE-LEZ (FR); TINCELIN, Christophe, 34000 MONTPELLIER (FR); LI, Tong, 75000 PARIS (FR)
(74) Representative: IPAZ

(57) **Abstract**

The invention relates to a portable handheld device (100) for measuring the force of a joint of a person of interest, POI, said device (100) comprising:
- a force measuring body (110), enclosed in a housing, comprising at least one connector (114) arranged on a side, called upper side, of said body for removably attaching to said body (110) an accessory (120;600) provided to be pressed towards said top side during for measurement; and
- a cup shaped bottom case (130) having a bottom surface and an open side opposite to said bottom surface, for removably receiving said body (110) with said upper side of the body (110) positioned on said open side, and provided to be in contact with a part of a body of said POI during force measurement.

## Description

### Field of the invention

The present invention relates to a portable handheld device for measuring the force of a joint of a person.

The field of the invention is the field of handheld devices for measuring the force of a joint of a person, also called device for measuring the force of a muscle of a person.

### Background

In various situations, handheld devices are needed for measuring the force of a person, and especially the force of a joint of a person. For example, practitioner, such as doctors or physiotherapist, need to measure the force of a joint of a person during a diagnosis, or a period of rehabilitation. These devices may also be used by trainers for measuring the performance of an athlete, and optionally monitoring said performance over time during a training period for example.

There are a variety of handheld devices for measuring the force of a joint, or of a muscle, of a person. In a non-limitative example of use, the measuring device is in contact with a part of the body of a person of interest under measurement, for example a thigh of said person of interest. The person of interest exerts a force against the device in a given direction while a third person, a practitioner or a trainer, exerts a pressure on said device in the opposite direction. A sensor is arranged in the device for measuring the force applied on said device.

Despite all efforts, current handheld devices are not user friendly and complicated to use. Plus, the usability of the current handheld devices is very limited, not to mention that they are not flexible to use.

A purpose of the present invention is to overcome at least one of these drawbacks.

Another purpose of the present invention is to provide a portable handheld device for measuring the force of a joint of a person that is more user friendly and more simple to use.

Another purpose of the present invention is to provide a portable handheld device for measuring the force of a joint of a person, the usability of which is improved.

Another purpose of the present invention is to provide a portable handheld device for measuring the force of a joint of a person, more flexible in use.

### Summary of the invention

The invention makes it possible to achieve at least one of these aims by a portable handheld device for measuring the force of a joint of a person of interest, POI, said device comprising:
- a force measuring body, enclosed in a housing, comprising at least one connector arranged on a side, called upper side, of said body for removably attaching to said body an accessory provided to be pressed towards said top side during for measurement,
- a cup shaped bottom case having an open side and a bottom surface opposite to said open side, for removably receiving said body on the opposite side to said upper side, and provided to be in contact with a part of a body of said POI during force measurement.

The present invention thus proposes portable handheld device for measuring the force of a joint of person comprising a force measuring body, also called body in the following, which is the main part of the device.

The device according to the invention also comprises a bottom case that is provided to be in contact with a part of the body of the POI. The invention proposes that the bottom case is attached in a removable fashion to the measuring body. Thus, the device according to the invention may be adapted to different parts of the body of the POI, by changing said bottom case. Thus the device according to the invention is simpler to use, but also more flexible and more ergonomic. Also, allowing the device according to the invention to adapt to different parts of the POI makes the device more usable because it can be used for measuring the force of different joints of the body of a POI. Moreover, the detachable bottom case allows easy cleaning of the device.

Also, according to the invention, an accessory may be attached in a removable manner to the measuring body, said accessory being provided to be pressed by a third person, such as a practitioner or a trained, during force measurement. Thus, the device according to the invention may be adapted to different third persons, and to different force measurement techniques, by changing the accessory. Thus, the device according to the invention is simpler to use, but also more flexible and more usable. Moreover, the detachable accessory allows easy cleaning of the device.

For these reasons, the device according to the invention provides more accurate measurements, is simpler to use, more flexible, more usable, and more ergonomic, compared to prior art devices.

In the present document, the terms "bottom" and "upper" are used to indicate relative relationships between the components of the device, seen from de point of view of the third person. A "bottom" side/surface is closer to the POI compared to the third person. Conversely, an "upper" side/surface is closer to the third person compared to the POI.

The device according to the invention may comprise at least one battery for providing electrical energy to said device.

At least one battery of the device may be placed in the measuring body.

At least one battery may be rechargeable.

The connector may be any type of mechanical connector,

For example, the connector may be a male-female type connector. The female part of the connector may be arranged on the body and the male part of the connector may be arranged on the accessory, and *vice versa.*

According to some embodiments, the connector may allow attachment of the accessory in a direction perpendicular to the upper side.

In other words, the connector may allow attachment of the accessory in the direction the pressure is applied to the accessory. Thus, an unintentional or undesired detachment of the accessory is prevented during use of the device according of the invention.

In some embodiments, the connector may allow attachment of an accessory at a first level not allowing rotation of said accessory around a direction perpendicular to the top side.

In this case, when the accessory is placed at said first level, the rotation of the accessory is prevented and a pressure may be applied to the accessory without a risk of twisting the accessory. This allows a more secured manipulation of the device.

In some embodiments, the connector may allow attachment of an accessory in a second level allowing rotation of said accessory around a direction perpendicular to the top side.

In this case, when the accessory is placed at said second level, the rotation of the accessory is authorised, making it possible to modify the orientation of the accessory relatively to the measuring body. This enhances the usability of the device according to the invention. For example, it allows a more flexible positioning of the hand of the third person, and consequently of the position of said third person, when exerting a pressure on said accessory.

The rotation of the accessory may be limited to a predetermined angle, such as 90°. Alternatively, the angle of rotation of the accessory may not be limited and the accessory may be rotated over 360°.

The rotation of the accessory may be authorised continuously so that the accessory may be placed at any desired angle. For example, if the rotation of the accessory is limited to 90°, the accessory may be authorized to be placed at any angle between 0° and 90°.Alternatively, the rotation of the accessory may be authorized so that the accessory may be placed only at predetermined discrete values. For example, if the rotation of the accessory is limited to 90°, the accessory may be authorized to be placed only at the following angles: 0°, 45°, 90°.

When the connector allows attachment of the accessory to a first level and a second level, the first level may be below the second level, in the direction the pressure/force is applied to the accessory, so that when a pressure is applied the accessory, said accessory is put in the first level of attachment preventing rotation of the accessory as long as a pressure is exerted on the accessory.

When the connector allows attachment of the accessory to several levels, the connector or the accessory may comprise a mean for positioning the accessory to one of said levels, for example to the first level, especially when no pulling is applied to said accessory and the accessory is at rest. For example, the positioning mean may comprise retaining means, or similar, positioning the accessory always to the first level. In this case, the accessory is, in particular always, placed in the first level when no pressure is exerted on said accessory and is in the second level when a pulling pressure is applied to the accessory.

In some embodiments, the device may comprise a retaining means for retaining the accessory with the body when no pressure is exerted on said accessory.

Thus the accessory is held with the measuring body even if a pressure is not applied to said accessory. This allows a more ergonomic and easier manipulation of the device according to the invention.

The retaining means may be arranged in the measuring body, and more particularly in the connector.

The retaining means may of any type.

For example, the retaining means may carry out magnetic retaining of the accessory. In this case, at least one magnet may be arranged in the measuring body, respectively in the accessory, said magnet cooperating with a magnet or a metallic member, arranged in said accessory, respectively said measuring body, for retaining the accessory secured to the measuring body.

The bottom case may be attached to the body by any attachment means, as long as the attachment is carried out in a removable fashion, especially manually, and more particularly without use of a tool for attaching and detaching said bottom case.

According to some embodiments, the attachment of the bottom case to the body may be carried out by magnetic attachment means, adhesive attachment means, by screwing, etc.

In some embodiments, the bottom case may be attached to the body by clipping. In this case, one or several clips may be provided on one of the bottom case and the body. When the body is inserted in the cup shaped bottom case, each clip lodges in a hole, or a recess, provided in the other one of the bottom case and the body. Thus the bottom case may be attached to the body manually, without use of a tool.

In some embodiment, the clip(s) may be provided on the bottom case, and especially at the level of the border of the open side of the bottom case. The recess(es) or hole(s) may thus provided on the housing of the body at corresponding locations.

According to some embodiments, the bottom case may comprise at least one detaching means allowing a user to detach the bottom case from the body, in particular manually, and more particularly without using any tool.

In some embodiments, the detaching means may comprise, or may be, a detaching leap, provided to be pulled for detaching said bottom case from the measuring body. Thus, the user holds the detaching lip and pulls on it in order to detach the bottom case from the body.

The detaching means, and in particular the detaching lip, may be placed at any location on the bottom case. In a particular and non-limitative example, the detaching means, and in particular the detaching leap, may be arranged at the level of the border of the open side of said bottom case. This facilitate the manipulation of the bottom case for detaching it from the body. Plus, the detachment of the bottom case from the body may be done with less effort from the user.

The bottom case may comprise a lateral face that is stiffer than the bottom face.

Thus, the bottom case may securely be attached to the body, while the bottom face, provided to be in contact with a part of a body of the POI, may be more ergonomic to use and more user friendly.

Of course, the device according to the invention may comprise only one bottom case.

In some embodiments, there might be provided, or the device according to the invention may comprise, several interchangeable bottom cases, that are removably attachable to the measuring body, one at a time. This allows adapting the form and the outer dimensions of the bottom case to a specific POI or to a specific body part of the POI, rendering the device according to the invention more flexible and more usable.

For example, at least one of the following characteristic of the bottom case may vary from one bottom case to another: the width, the length, the height, the overall form, the form of the bottom surface, the type of the material, the stiffness and elasticity, etc.

For example, at least one bottom case may have a concave bottom surface allowing better positioning on thighs of a POI, whereas another bottom case may have a generally flat bottom surface.

Of course, the device according to the invention may comprise only one accessory.

In some embodiments, there might be provided, or the device according to the invention may comprise, several interchangeable accessories, that are removably attachable to the measuring body, one at a time. This allows adapting the accessory to a specific practitioner, or to a specific exercise, or to a specific force measurement technique, rendering the device according to the invention more flexible and more usable.

For example, at least one of the following characteristic of the accessory may vary from one accessory to another: the width, the length, the height, the overall form, the type of the material, etc.

For example, at least one accessory may have a generally flat form, whereas another accessory may have one or several handles to be hold by the practitioner during force measurement. Of course other types of accessories may be used.

In some embodiments, the device according to the invention may further comprise at least one command button arranged on the top side of the force measuring body.

Thus, the device according to the invention allows access to said command button during force measurement, and more general during use of the device.

The command button may be used to command at least one function, such as at least one the following functions: starting/stopping the device, changing an exercise, changing a force measurement technique, triggering data transmission from the device to an external apparatus, etc.

In some embodiments, the device according to the invention may further comprise a display means arranged on the top side of the force measuring body.

Thus, the device according to the invention allows access to said display during force measurement, and more general during use of the device.

The display may comprise at least one led, or at least one screen.

The display means may display information/data regarding the status of the device, notifications regarding the device, notifications regarding the POI, notifications regarding the measurement force, etc.

The device according to the invention may further comprise an electrical interface.

Advantageously, the electrical interface may be arranged on the top side of the force measuring body, and more particularly on the top surface of the force measuring body. Thus, the electrical interface is accessible any time, especially during use of the device.

The electrical interface may be of any type. Preferably, the electrical interface may be an electrical port, and especially a USB port.

The electrical interface may be used for charging the device, and especially a battery of the device.

Alternatively, or in addition, the electrical interface may be used for wired communication between said device and an external apparatus, such as a computer, a tablet, a server, etc.

Of course, the measuring body comprises a force sensor for measuring the force exerted by the POI on said device, i.e. on the bottom case that is in contact with a part of the body of the POI.

Such a sensor is known and its architecture in the device may be done according to known techniques. Such a sensor may use a measuring technique for example strain gauge resistive electric sensors, or capacitive load sensors The architecture may be that of any type, preferably that of a bending beam, or compression body.

The measuring body may also comprise an inertia sensor, and/or an angle sensor, and/or an accelerometer.

Such sensor(s) allow controlling and proper positioning the measuring device during use, enhancing the usability of the device according to the invention.

Plus, such sensor(s) allows efficient and proper use of the device according to the invention for measurement(s)/exercice(s) involving movement from the POI and/or the third person, such as isokinetic measurement(s)/exercise(s).

Such sensor(s) also allow controlling and proper positioning the measuring device when the POI has to exert pressure at a given angle.

### Description of the figures and embodiments

Other advantages and characteristics will become apparent on examination of the detailed description of an embodiment which is in no way limitative, and the attached figures, where:
- FIGURES 1-3 are diagrammatic representations of a non-limitative example of a device according to the invention;
- FIGURE 4 is a diagrammatic representation of an example of an accessory that may be used with a device according to the invention;
- FIGURES 5a-5c are diagrammatic representations of non-limitative examples of bottom cases that may be used with a device according to the invention;
- FIGURE 6 is a diagrammatic representation of a non-limitative example of an accessory that may be used with a device according to the invention;
- FIGURE 7 is a diagrammatic representation of a non-limitative example of a device according to the invention; and
- FIGURES 8a and 8b are diagrammatic representations of the non-limitative example of FIGURES 1-3.

It is well understood that the embodiments that will be described below are in no way limitative. In particular, it is possible to imagine variants of the invention comprising only a selection of the characteristics described hereinafter, in isolation from the other characteristics described, if this selection of characteristics is sufficient to confer a technical advantage or to differentiate the invention with respect to the state of the prior art. Such a selection comprises at least one, preferably functional, characteristic without structural details, or with only a part of the structural details if this part alone is sufficient to confer a technical advantage or to differentiate the invention with respect to the prior art.

In the FIGURES, elements common to several figures retain the same reference.

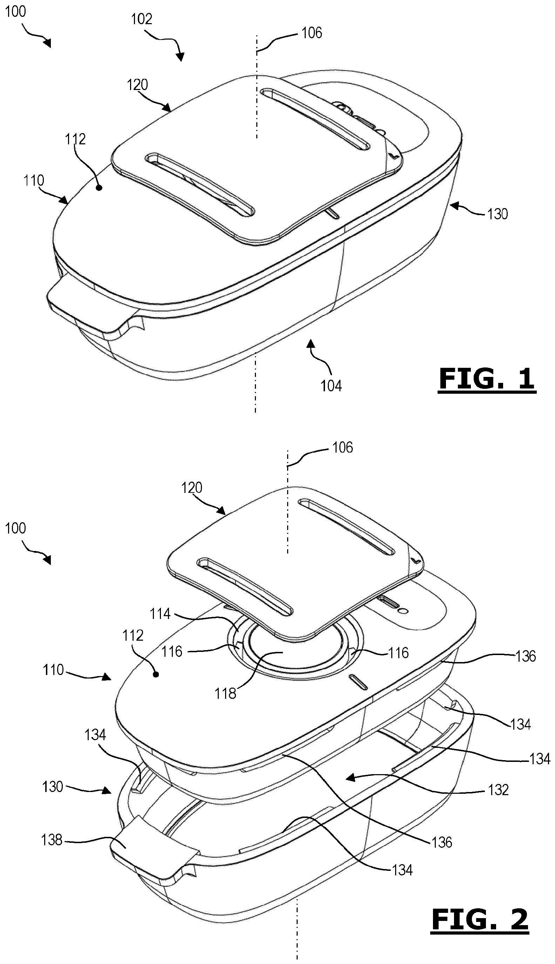

FIGURES 1-3 are diagrammatic representations of a non-limitative example of a device according to the invention, according to different points of view.

FIGURE 1 is a diagrammatic representation of the device according an isometric view.

FIGURE 2 is a diagrammatic representation of the device in an exploded isometric view.

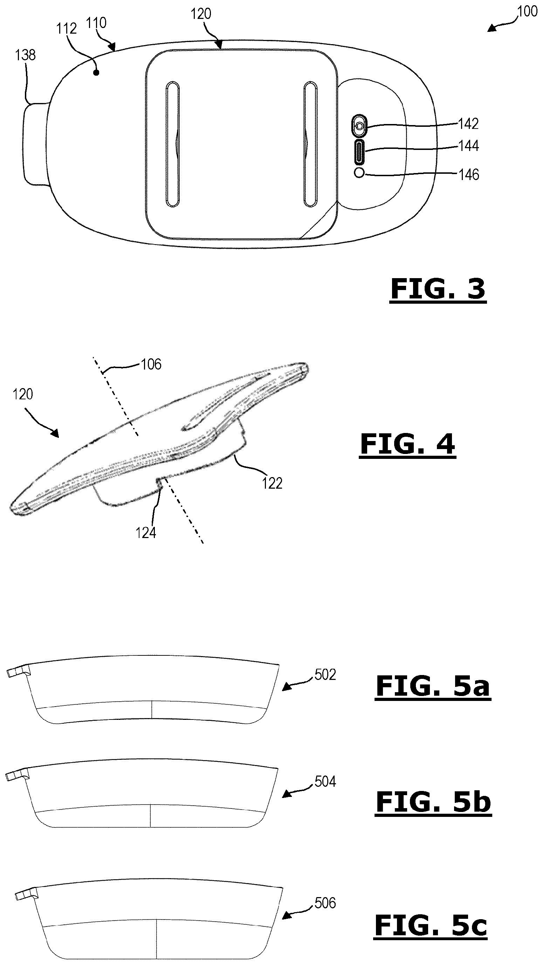

FIGURE 3 is a diagrammatic representation of the device, from the top side of the device.

The device 100, shown in FIGURES 1-3, may be used for example for measuring the force of a joint of a person of interest, POI, such as a knee, an elbow, an ankle, a hip, a wrist, a shoulder, etc.

The device 100 comprises a first side 102, called top side, and a second side 104, called bottom side, opposite to the top side 102. In use, the bottom side 104 of the device is placed on a part of the body of a person of interest, POI, also called a user. A third person, such as a practitioner or a trainer, exerts a pressure on the top side 102 of the device 100, for example in a direction 106 perpendicular to the device 100, or to the top side 102. The POI applies an opposing force on the bottom side 104 of the device 100 in order to balance the pressure applied by the third person on the top side 102. The force applied by the POI is thus measured and corresponds to the force applied by a joint of said POI in contact with the bottom side 104. For example, if the body part in contact with the bottom side 104 is a shin of the POI, the joint of the person for which the force is measured may be the knee of the person.

The device 100 of FIGURES 1-3 comprises a force measuring body 110, also called body in the following, comprising components for measuring the force of a joint of a POI. For example, the measuring body 102 comprises force measuring sensor(s), a battery for supplying electrical energy to the device 100, an electronic board, etc. These components are not shown in the FIGURES since they are standard components.

The body 110 comprises a top face 112. An accessory 120, as it will be described further, is intended to be attached to the body 110, in a removable way, at the side of the top face 112. Thus, the body 110 comprises, on the side of the top face 112, a connector for attaching said accessory to the body 110, in a removable manner. In the example shown in FIGURES 1 and 2, the connector comprises a trench 114 provided to receive a lip that is provided on the accessory. In a non-limitative example, the trench 114 may be a circular trench as shown in FIGURE 2. Of course the trench 114 may have another form. In other embodiments, the connector provided on the body 110 may not comprise a trench. For example, the connector may comprise clip(s), hole(s), etc.

The body 110 may also comprise retaining means for retaining the accessory 120 attached to the body 110, when said accessory 120 is connected to the body 110. In some examples, the retaining means may comprise one or several magnets (not shown) arranged in/on the body 110 and provided to cooperate with magnet(s), or metallic members, arranged in/on the accessory 120 for retaining the accessory 120 with the body 110 when said accessory 120 is attached to said body 110.

Moreover, the body 110 may comprise means for authorising rotation of the accessory 120 with respect to the body 110, especially around the direction 106. Thus, the accessory 120 may be placed in different configurations, rendering the device 100 according of the invention more flexible and more usable. The rotation of the accessory 120 may be limited to a predetermined angle, such as 90°. Alternatively, the angle of rotation of the accessory 120 may not be limited and the accessory may be rotated over 360° around the direction 106. The rotation of the accessory 120 may be authorised continuously so that the accessory 120 may be placed at any desired angle. For example, if the rotation of the accessory 120 is limited to 90°, the accessory 120 may be authorized to be placed at any angle between 0° and 90°.Alternatively, the rotation of the accessory 120 may be authorized so that the accessory 120 may be placed only at predetermined discrete values. For example, if the rotation of the accessory 120 is limited to 90°, the accessory 120 may be authorized to be placed only at the following angles: 0° and 90°.

In the example of FIGURES 1-3, without loss of generality, the accessory 120 may be connected to the body 110 and authorised to be positioned at two different angular positions around the direction 106, for example separated by an angle of 90°. To allow positioning of the accessory in these different angular positions, the trench 114 comprises holes 116 arranged at the bottom of the trench 114. Each hole 116 is intended to receive a tooth provided on the accessory 120 when the accessory is in an angular position. For example, holes 116 may be arranged so that at least one of them receives a tooth provided on the accessory 120 when the accessory is in a first angular position corresponding to a 0° angle, another tooth one in a second position corresponding to a 90° angle. Of course, other means than those described above may be used to allow positioning of the accessory in different angular positions with respect to the body 110. For example, holes 116 may be replaced by grooves and the teeth may be replaced by wedges. In other examples, the accessory 120 may be screwed and thus allowed to be positioned at different angular positions. The invention is thus not limited to the specific example.

. At rest, i.e. when no solicitation is applied to the accessory 120, the accessory is attracted/pulled towards the body 110, for example by the retaining means, i.e. magnet(s) placed in the potion 118, to a level, called first level, so that the accessory 120 is not authorized to rotate. When a solicitation in the form of a pulling force is applied to the accessory 120, the accessory 120 is placed into a level, called second level, at which the accessory 120 may be rotated For example, in this first level of attachment, the tooth/teeth provided on the accessory 120 may engage in the hole(s) 116 and the accessory locked in position around the direction 106. Here again, the invention is not limited to the use of a spring. For example, the rotation of the accessory 120 may be authorized by pulling said accessory 120 slightly away from the body.

As indicated above, in some embodiments, the retaining means, i.e. the magnet(s), may be arranged inside the portion 118.

The device 100 of FIGURES 1-3 may further comprise an accessory 120, as shown in FIGURES 1-3.

The accessory 120 is attached to the body 110, in a removable manner, so that said accessory 120 may be detached from the body manually, and more particularly without use of a tool. Thus the accessory 120 may be changed and replaced easily in order to adapt the device 100 to specific exercise(s) or force measurement technique(s), or in order to clean the accessory 120, or for maintenance, etc.

The accessory 120 is intended to be manipulated by, directly or indirectly, by a third person, such as a practitioner or a trainer.

FIGURE 4 shows a non-limitative example of an accessory that may be used with a device according to the invention, and more particularly the accessory 120 equipping the device of FIGURES 1-3.

The accessory 120 comprises a connector for attaching said accessory 120 to the body 110, on the top side of the body 110. In the example shown, and without loss of generality, the connector comprises a lip 122 engaging in the trench 114 provided on the top side 112 of the body 110. The lip 122 has a form that is complementary to the form of the trench 114. In the example shown, the lip 122 has a generally circular form. Of course, the invention is not limited to a connector in the form of lip. The accessory 120 may comprise other types of connectors, such as a threaded bore, a screw type member, a male or a female connector, clip(s), a scratch, a suction cup, etc. as long as it is adapted to attach, in a removable fashion, the accessory 120 to the measuring body 110.

Moreover, in the example shown, the accessory 120, and more particularly the lip 122, has at least one tooth 124 provided to engage with the hole(s) 116 provided at the bottom of the trench 114 in order to place the accessory 120 in different angular positions. Again, the invention is not limited to the use of a tooth on the accessory 120 and other means may be used for carrying out the same function.

The device 100 also comprises a bottom case 130, on the bottom side 104 of the device 100.

The bottom case 130 is intended to be in contact with the body of the POI during force measurement.

The bottom case 130 is attached to the body, in a removable manner, so that said bottom case 130 may be detached from the body 110 manually, and more particularly without use of a tool. Thus the case 130 may be changed and replaced easily in order to adapt the device 100 to specific exercise or force measurement technique, or in order to adapt the device 100 to a specific body part of the POI, or in order to clean the case, or for maintenance, etc.

The bottom case 130 is intended to receive at least the bottom part of the body 110.

In the example shown, the bottom case 130 has a generally cup shaped form, with an open side, and defines an inner space 132 for receiving at least the bottom part of the body 110. Thus, when attached to the body 110 the bottom case 130 covers at least part of the body 110. In the non-limitative example shown on FIGURES 1-3, the bottom case 130 receives the bottom and the lateral portion of the body 110, and envelops the body 110 until the edges of the top surface 112 of the body 110, as shown in FIGURE 1.

As indicated above, the bottom case 130 is attached to body 100 in a removable way. Such an attachment may be done in various ways, for example by magnetic attachment means, by suction attachment means, by velco^{®} type attachment means, etc. In the non-limitative example shown in FIGURES 1-3, best seen in FIGURE 2, the bottom case 130 is clipped to the body 110. More particularly, the bottom case 130 comprises several clips 134, for example placed at the level of the upper edge of the bottom case 130.Each of the clips is intended to engage with a recess 136 provided on the body 110, for example placed at the level, or just under, the edge of the upper face 112 of the body 110. Of course, alternatively, the clips 134 may be placed on the body 110 and the recesses 136 may be placed on the bottom case 130. Thus, the body 110 is inserted in the inner space 132 of the bottom case 130 and a slight pressure is applied on the body 110, and/or the bottom case 130, for engaging the clips 134 in the recesses 136, in order to attach the bottom case 130 to the body 110.

According to an advantageous feature, the bottom case 130 may comprise a lateral face that is stiffer than its bottom face. Thus, the bottom case 130 may securely be attached to the body, while the bottom face, provided to be in contact with a part of a body of the POI, may be more ergonomic to use.

In some embodiments, the bottom case 130 may comprise at least one detaching means allowing a user to detach the bottom case 130 from the body 110, in particular manually, and more particularly without using any tool.

In the non-limitative example shown in the FIGURES 1-3, the detaching means comprises a detaching lip 138 provided to be pulled for detaching the bottom case 130 from the body 110. Thus, the user holds the detaching lip 130 and pulls on it in order to detach the bottom case 130 from the body. By pulling the detaching lip 130, the clips 13 disengage from the recesses 136 and the bottom case 130 may be removed.

The detaching means, and in particular the detaching lip 138, may be placed at any location on the bottom case 130. In a particular and non-limitative example, as shown in the example, the detaching leap 138 is arranged at the level of the border of the open side the bottom case 130. This facilitates the manipulation of the bottom case 130 for detaching it from the body 110. Plus, the detachment of the bottom case 130 from the body 110 may be done with less effort from the user.

Alternatively, or in addition, a detaching means may be placed on the body 110.

The device 100 also comprises a command button 142 arranged on the top surface 112 of the body 110. Thus the command button 142 is accessible any time when the device 100 is in use.

The command button142 may be used to command at least one function, such as at least one the following functions: starting/stopping the device, changing an exercise, changing a force measurement technique, triggering data transmission from the device to an external apparatus, etc.

The device 100 further comprises a USB port 144 arranged on the top surface 112 of the body 110. Thus, the USB port 144 is accessible at any time, especially during use of the device 100.

The USB port may be used for charging the device 100, and especially a battery of the device 100. Alternatively, or in addition, the USB port may be used for wired communication between said device 100 and an external apparatus, such as a computer, a tablet, a server, etc. for data transmission.

In the non-limitative example shown in FIGURES 1-3, the device 100 also comprises a light indicator 146, such as a led. The light indicator 146 is placed on the top surface 106 so that the user has visual access to said indicator at any time, and especially during use of the device 100.

The light indictor 146 may display information/data regarding the status of the device 100, the status of the battery of the device 100, notifications regarding the device 100, notifications regarding the POI, and/or notifications regarding the measurement force, etc.

As indicated above, the device comprises a bottom case that is attached to the body in a removable manner so that it may be changed easily in order to adapt the device to the POI. For example, several bottom cases may be used interchangeably with the device according to the invention, one at a time.

At least two bottom cases may have different sizes, i.e. different heights, different widths, different lengths, different forms, different bottom surfaces, etc. Thus the device according to the invention may be adapted to different body parts of the POI.

At least two bottom cases may be made of different material(s).

FIGURES 5a-5c are diagrammatic representations of non-limitative examples of bottom cases that may be used with the device according to the invention.

The bottom cases 502-506, shown on the FIGURES 5a-5c may be used with any device according to the invention, and more particularly with the device 100 of FIGURES 1-3.

FIGURES 5a-5c show three different bottom cases 502-506.

The case 502 differs from the cases 504 and 506 by its bottom surface. The bottom surface of the case 502 is flat, whereas cases 504 and 506 have an incurved bottom surface.

The case 506 differs from the cases 504 and 502 by its external height. The case 506 has an external height that is greater than the external height of the cases 504 and 502.

Or course, these are only non-limitative examples. At least two bottom cases may be identical. Alternatively, or in addition, at least two bottom cases may differ from each other, for example may have different architectures, sizes, materials, etc.

As indicated above, the device may comprise an accessory that is attached to the body in a removable manner so that it may be changed easily in order to adapt the device to a third person, such as a practitioner, a trainer, etc. or to a force measuring exercise/technique. For example, several accessories may be used interchangeably with the device according to the invention, one at a time.

At least two accessories may differ from one another, for example may have different sizes, i.e. different heights, different width, different lengths, different forms, etc. Thus the device according to the invention may be easily personalized/adapted.

At least two accessories may be made of different material(s).

FIGURE 4, described above, shows a first non-limitative example of an accessory 120 that may be used with a device according to the invention.

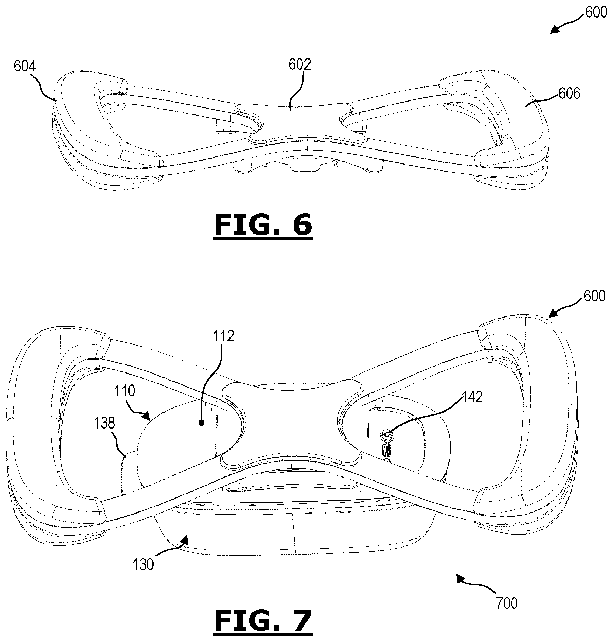

FIGURE 6 shows another non-limitative example of an accessory that may be used with a device according to the invention.

The accessory 600 of FIGURE 6 may be used with a device according to the invention, and more particularly with the device 100 of FIGURES 1-3.

The accessory 600 is larger than the accessory 120 and has a center portion 602 for attaching it to the body of the device. The accessory 600 also has handles 604 and 606, at each side of the center portion 602, so that it may be handed more easily by a POI or a third person, during use of the device. The handles 604 and 606, and thus the accessory 600, allow exerting a higher pressure compared to the accessory 120 for example.

FIGURE 7 shows another non-limitative example of a device according to the invention.

The device 700 of FIGURE 7 comprises all the components of the device 100 of FIGURES 1-3, except that the accessory 120 is replaced by the accessory 600 of FIGURE 6, in the device 700.

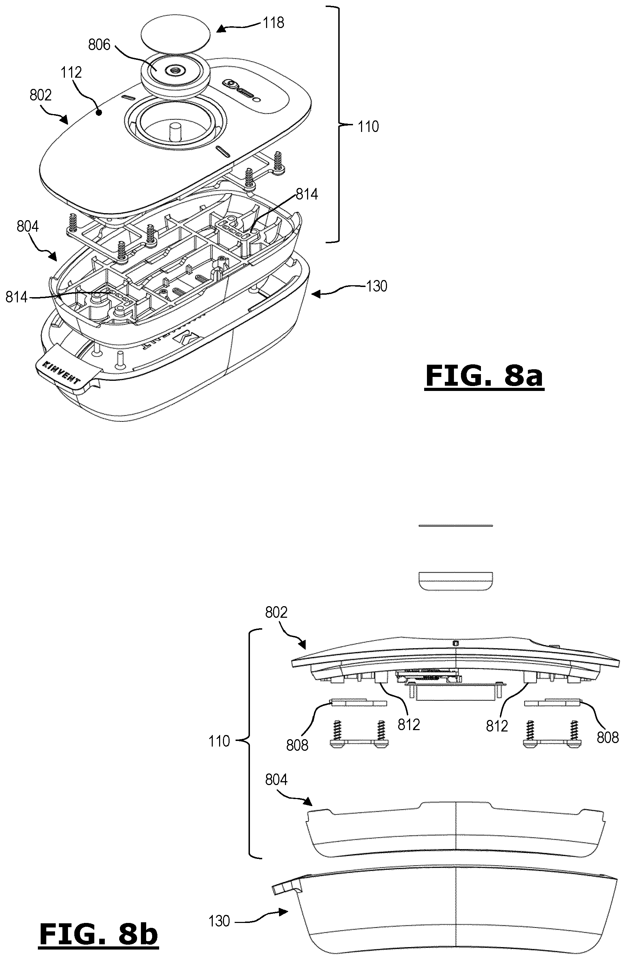

FIGURES 8a and 8b are diagrammatic representations of the non-limitative example of FIGURES 1-3.

More particularly, FIGURE 8a shows the device 100 of the FIGURE 1-3 in a further exploded isometric view and FIGURE 8b shows the device 100 of the FIGURE 1-3 in a further exploded lateral view.

As shown in the non limitative example of FIGURES 8a and 8b, the measuring body 110 is composed of a first part 802, called upper part, in contact with the accessory 110 and a second part 804, called bottom part, in contact with the bottom case 130. The upper part 802 and the bottom part 804 are assembled together and define an interior space in which are placed the components of the measuring body 110.

FIGURE 8a shows the retaining means, and especially a magnet 806 acting as retaining means, placed in the portion 118. This magnet exerts a pulling force on a magnet or a metallic piece placed in the accessory 110 so that the accessory is retained with the measuring body 110.

Moreover, FIGURE 8b shows that the device 100 comprises two sensors 808 et 808 for measuring the force applied to the device 100. Each sensor 808 may be any type of force measuring sensor such as strain gauge, or capacitive sensor, etc.

Each sensor 808 is placed inside the measuring body 110. More particularly, each sensor 808 is mounted to the upper part 802 of the measuring body 110 thanks to mounting pots 812 provided on said upper part 802, inside the measuring body 110. Each sensor 808 is also in physical contact with surfaces, or forms or pins, provided on the bottom part 804 of said force measuring body 110. Thus, each sensor 808 is sandwiched between the upper part 802 and the bottom part 804, and in physical contact with said upper part 802 and said bottom part 804.

A force applied on the accessory 110 and/or on the bottom case 130 is transferred the measuring body 110, because said accessory 110 and said bottom case 130 are in physical contact with, respectively the upper part 802 and the bottom part 804 of the measuring body. Thus, the applied force is transferred to the upper part 802 and to the bottom part 804 of the measuring body 110. Since each sensor is in contact with said upper part 802 and bottom part 804, and is sandwiched between said upper part 802 and bottom part 804, the applied force is transferred to said force measuring sensor 808 so that the latter measures said applied force.

Of course, the example shown on FIGURES 8a and 8b is a non limitative example. Other force measuring architectures may be used within the measuring body 110.

Of course, the invention is not limited to the examples detailed above.

## Claims

1. A portable handheld device (100;700) for measuring the force of a joint of a person of interest, POI, said device (100;700) comprising:
- a force measuring body (110), enclosed in a housing, comprising at least one connector (114) arranged on a side, called upper side, of said body for removably attaching to said body (110) an accessory (120;600) provided to be pressed towards said top side during for measurement; and
- a cup shaped bottom case (130;502-506) having an open side and a bottom surface opposite to said open side, for removably receiving said body (110) on the opposite side to said upper side, and provided to be in contact with a part of a body of said POI during force measurement.

2. The device (200;700) according to the preceding claim, **characterized in that** the connector (114) allows attachment of the accessory in a direction perpendicular to the upper side.

3. The device (100;700) according to any one of the preceding claims, **characterized in that** the connector (114) allows attachment of an accessory (120;600) at a first level not allowing rotation of said accessory around a direction perpendicular to the top side.

4. The device (200;700) according to any one of the preceding claims, **characterized in that** the connector (114) allows attachment of an accessory (120;600) in a second level allowing rotation of said accessory (120;600) around a direction (106) perpendicular to the top side.

5. The device (100;700) according to any one of the preceding claims, **characterized in that** it comprises a retaining means for retaining the accessory (120;600) with the body (110) when no pressure is exerted on said accessory (120;600).

6. The device (100;700) according to any one of the preceding claims, **characterized in that** the bottom case (130;502-506) is attached to the body (110) by clipping.

7. The device (100;700) according to any one of the preceding claims, **characterized in that** the bottom case (130;502-506) comprises at least a detaching leap (138), in particular arranged on a border of the open side of said bottom case (130;502-506), and provided to be pulled for detaching said bottom case (130;502-506) from the measuring body (110).

8. The device (100;700) according to any one of the preceding claims, **characterized in that** the bottom case (130;502-506) comprises a lateral face that is stiffer than the bottom surface.

9. The device (100;700) according to any one of the preceding claims, **characterized in that** it comprises several interchangeable bottom cases (130;502-506) that are removably attachable to the measuring body (110), one at a time.

10. The device (100;700) according to any one of the preceding claims, **characterized in that** it also comprises at least one accessory (120;600) removably attaching to the measuring body (110).

11. The device (100;700) according to any one of the preceding claims, **characterized in that** it further comprises at least one command button (142) arranged on the top side of the force measuring body (110).

12. The device (100;700) according to any one of the preceding claims, **characterized in that** it further comprises an electrical interface (144) arranged on the top side of the force measuring body (110).

13. The device (100;700) according to any one of the preceding claims, **characterized in that** it further comprises a display means (146) arranged on the top side of the force measuring body (110).

14. The device (100;700) according to any one of the preceding claims, **characterized in that** the measuring body (110) comprises a force sensor, and at least one of the following sensors: an inertia sensor, and/or an angle sensor, and/or an accelerometer.
